# EUROPEAN PATENT SPECIFICATION

(11) **EP 0 619 993 B1**
(45) Date of publication and mention of the grant of the patent: **02.07.1997**
(21) Application number: 94301717.8
(22) Date of filing: 10.03.1994
(51) Int. Cl.: A61F 9/00

(54) **A surgical instrument for use in opthalmic surgery**
Augenchirurgische Vorrichtung
Appareil chirurgical opthalmique

(30) Priority: 12.03.1993 ZA 931767; 26.07.1993 US 98082
(43) Date of publication of application: 19.10.1994
(73) Proprietor: Amoils, Selig Percy, Athol, Sandston 2196 (ZA)
(72) Inventor: Amoils, Selig Percy, Athol, Sandston 2196 (ZA)
(74) Representative: Allsop, John Rowland

(56) References cited:
- DE-A- 2 065 681
- DE-A- 2 242 863
- DE-A- 3 826 414
- FR-A- 2 302 713
- US-A- 3 952 732
- US-A- 5 069 664

## Description

This invention relates to surgical implements and techniques, and more particularly such instruments and techniques as used in ophthalmic surgery.

In certain ophthalmic techniques it is necessary to remove the nucleus and cortex of a lens. This technique is required most particularly for cataract operations.

Currently the most advanced method of removal of the nucleus is by phaco-emulsification, (a technique disclosed in US Patent No 3,589,363 - Banko and Kelman, and also in Phaco-emulsification Surgery, Devine et al, Permagon Press, 1991, pages 1-5). US-A-3 589 363 discloses all the features of the preamble of claim 1. In this technique, a probe is used which comprises a hollow tip (needle) that is oscillated longitudinally by a transducer which is either a stack of metal plates (a magneto-strictive driver) or crystal (piezoelectric) or an acoustic vibrator (ultrasonic). A small incision is made in the cornea and the tip of the phaco-emulsifier is inserted through the incision into the capsular membrane which supports the cloudy lens. The tip is brought into contact with the nucleus and is oscillated at high acoustic and ultrasonic frequencies between 27 and 64 kHz with amplitude of between 50 and 60 microns. The tip will break up the hard nucleus of the lens into tiny particles which can be sucked up or aspirated through the tip. This technique which is widely used is known as "small incision surgery" or "phaco surgery", and it enables saving the lens envelope or capsule, after the nucleus and surrounding cortex are removed, for the insertion of a plastic or other substitute or replacement lens.

The technique does, however, have certain disadvantages. First, the longitudinal or normal hammering effect of the oscillating tip tends to move the nucleus away from the tip and in particular to push from the immediate vicinity of the tip the particles that are broken away from the nucleus. Thus a very strong vacuum (order of 100mm or more) is required both to hold the nucleus against the hammering tip and to suck such particles through the tip. Second, there is a danger that the tip itself or the particles broken up thereby may penetrate the capsule. This will cause complications during the operation as the jelly within the eye ball will tend to escape through an opening so made.

In accordance with the discovery underlying the present invention, by replacing the longitudinal normal hammering action with an oscillating lateral, arcuate or rotary cutting or fragmenting action upon the nucleus, all of the above problems have been found to be obviated, including the relaxing of the requirement for the rather strong holding vacuum, as distinguished from merely aspirating the cut nucleus fragments.

An object of the present invention, accordingly, is to provide a new and improved surgical apparatus or instrument for fragmenting the nucleus of the eye lens under small incision or "phaco" surgery, that admirably obviates the above-described disadvantages and problems with current hammering techniques, provides considerably improved safety, and if desired, enables reduced vacuum requirements -- such being effected through the use of rotary oscillation cutting or fragmenting of the nucleus, as opposed to prior art longitudinal hammering action.

According to the invention there is provided a phaco-surgical ophthalmic lens nucleus fragmenting instrument comprising a longitudinally extending probe having a hollow tubular cutting tip extending along the longitudinal axis thereof, means for setting the tip into ultrasonic oscillation, means for controlling the oscillation intermittently, and means for applying an aspiration vacuum to the hollow of the tip during oscillation, characterised in that said setting means provides rotary oscillation to rotate the tip over a small angle in a plane transverse to the longitudinal axis of the probe and tip.

By incorporating a rotary oscillating device as defined, the needle tip is able to rotate back and forth or in transversely or lateral planes, as distinguished from longitudinal normal hammering, and within a very small angle (which action is hereinafter described by the generic term "rotary oscillation") and at high acoustic to ultrasonic speeds so that the tip can make successive lateral or somewhat arcuate or partially circular cuts into the hard nucleus -- a transverse or shearing ultrasonic frequency-driven fragmentation, as distinguished from prior art longitudinal hammering or chipping. While back-and-forth small arcuate cutting - fragmenting is preferred, in some instances, more complete rotary oscillation or helical or cork-screw type rotary oscillation may also be considered.

The end of the tip is provided with a cutting edge and this may be conveniently serrated for improved cutting ability. The rotary oscillation of the tip is preferably, though not exclusively, affected by means of ultrasonic crystals which act on a lever to effect the rotary oscillation of the tip. The tip end may be sharpened to improve destruction of the hard nucleus. This sharpened tip may also, as above stated, be serrated for improved cutting ability.

Furthermore, the tip may also be subject to a slight longitudinal linear oscillation (substantially normal) component in addition to the lateral or rotary movement referred to above.

A preferred and best mode embodiment of the invention will now be described by way of example, with reference to the accompanying drawings, wherein:
Figure 1 is an enlarged diagrammatic view of the eye of a patient being treated by the apparatus and technique of the invention;
Figures 2 and 3 are respectively a longitudinal and a transverse section through a phaco-"emulsifier" or fragmenter of the invention; and
Figures 4 and 5 are views similar to Figures 2 and 3, respectively, of a modification.

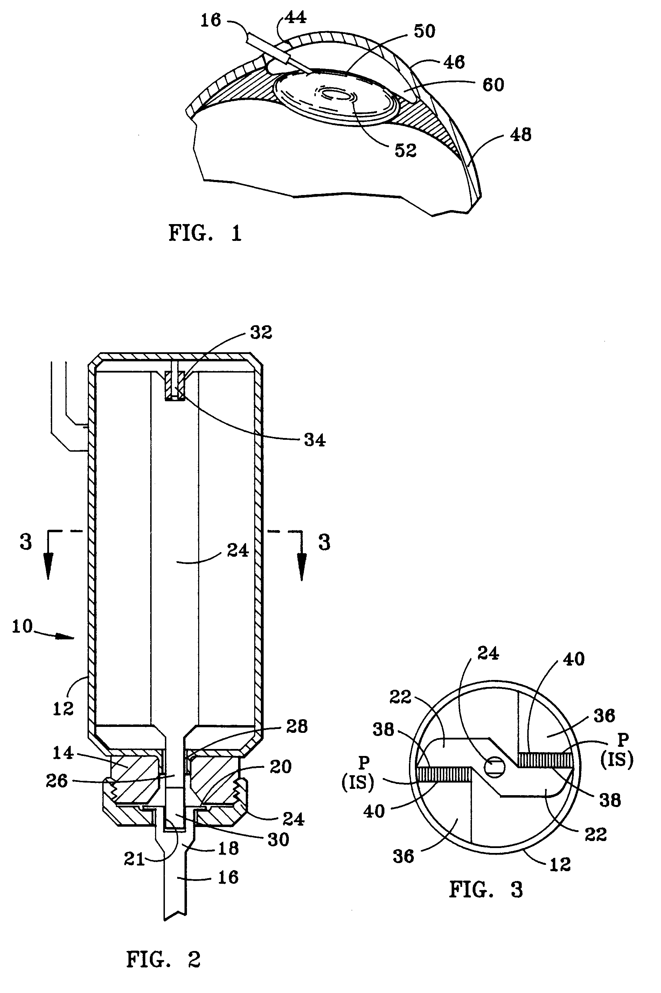

Referring now to Figures 2 and 3, there is shown a phaco-"emulsifier" or fragmenter 10 comprising a "Teflon®" or similar plastic cylindrical longitudinally extending body 12 that is dimensioned such that it can be easily carried and manipulated by a surgeon. The body 12 has an outlet neck 14 from which projects a probe having a tubular tip (needle-like) 16 of approximately 1.5 mm inside diameter and 0.25 mm wall thickness extending along the longitudinal axis of the probe. The distal end of the tip 16 (which is not shown) is sharpened and is preferably serrated. The inner end of the tip 16 has an enlarged portion 18 with a flange 20 and a non-circular, preferably square, recess 21 for a purpose later described. A cap 24 having a central aperture through which the portion 18 projects is threaded on to the neck 14 to hold the flange 20, and through it the tip 16, firmly on the neck in such a way as to be rotatable thereon.

Within the body 12 there is provided a generally "Z" - shaped preferably titanium core 22, Figure 3, which carries a centre shaft 24. The shaft 24 has an end projection 26, Figure 2, that passes through a hearing 28 at the neck 14 and has a non-circular end 30 that engages in the recess 21 to transfer rotary motion thereto. An internal bearing 32 runs on a centre pin 34 at the rear end of the housing 12 to provide additional support to the shaft 24.

A pair of elongated "Teflon®" suspension members 36, Figure 3, run longitudinally and internally of the body 12. The electrode members 36 carry respectively a pair of ultrasonic crystals 38 against which the end side faces 40 of the arms of the core 22 butt, with the respective arms of the core serving as ground electrodes. The crystals 38 are connected by the electrode to a source of electric power, schematically represented at P, to cause them to oscillate alternately and/or in synchronism and at intermittent intervals under a conventional foot pedal or other on-off switch schematically represented at IS, Figure 3, necessary as the surgeon progressively fragments the nucleus. This oscillatory movement is transferred to the core 22 causing it to rotate back and forth through a very small acute angle, which may be of the order of 5°, at high ultrasonic speed which may be of the order of 25 to 55 kHz, and in a plane transverse to the longitudinal axis of the probe and tip.

A well known surround (not shown) is provided around the proximal end of the tip 16 and is connected to an aspirator whereby suction of the order of 20 mm Hg can be applied to the centre tip 16 to withdraw material at the end of the tip 16.

The phaco-"emulsifier" or fragmenter 10 is intended to be used for the removal of cataracts in small incision surgery of phaco surgery. In such surgery, a small round incision 44 (see Figure 1) which may be as small as 3 mm in diameter, is made in the cornea 46 of the eye 48 near the limbus and a portion of the capsule 50 of the lens 52 is removed. The tip 16 of the instrument probe is inserted into the incision through the opening in the capsule 50 and the cortex therewithin and into the engagement with the hard nucleus 52. The rotary or back-and-forth rocking oscillation of the tip 16 enables it transversely or laterally to cut into the successive layers of the hard nucleus as the instrument is pressed by the surgeon there-against in the longitudinal axis direction, breaking off pieces or portions of the successive layers of the hard nucleus which can be withdrawn from the site of the operation by the fluid aspirated under vacuum applied in the hollow of the tip, as is well-known. After the nucleus has been thus broken down and removed from the capsule, the cortex surrounding the nucleus can be aspirated by the vacuum applied to the tip or using a separate aspiration hand piece. The eye is now ready to receive the replacement lens.

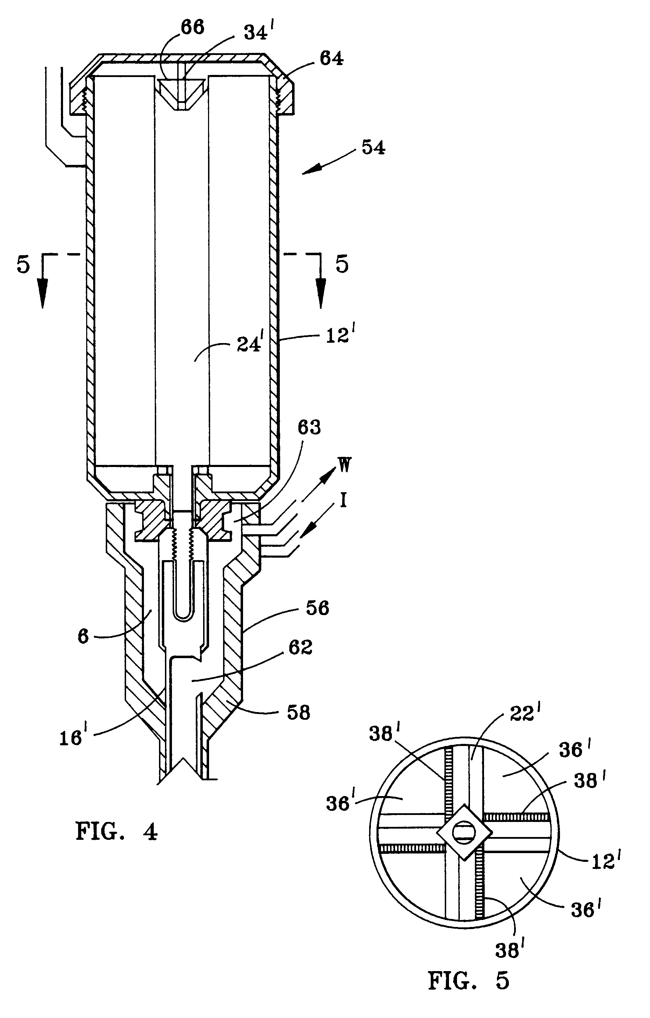

Reference is now made to Figures 4 and 5. The phaco-"emulsifier" or fragmenter 54 here shown differs from that of Figures 2 and 3 in that four crystals 38, Figure 5, are provided mounted respectively upon four suspension electrode members 36'. The core 22' is of a correspondingly cruciform shape. Furthermore the tip 16', Figure 4, is encased in a cylindrical surround 56 having an annular passage 58 through which a saline fluid can be introduced at I into the anterior chamber of the eye 60, Figure 1, and withdrawn at W through the tip 16'. This same construction may be used in the embodiment of Figures 2 and 3 as well. An opening 62 is provided in the proximal end of the tip 16' leading to an enlarged chamber 63 near the rear end of the surround 56.

The rear end of the body 12' of the phaco-"emulsifier" 54 is constituted by the removable cap 64 that is screw threaded on to the body 12'. The cap carries the centre pin 34' on which is mounted a conical bearing 66 carrying the rear end of the shaft 24'.

The phaco-"emulsifier" 54 is used in the same way as the phaco-"emulsifier" 10 of Figures 2 and 3.

I have found that the phaco-"emulsifiers" or fragmenters 10 and 54, as above described, have a number of advantages. First, the rotary oscillation does not have the "hammering" effect of the linear oscillating phaco-"emulsifier" of the prior art which tends to move the nucleus and or fragmented parts of the nucleus which are broken off away from the tip. Consequently, lesser vacuum pressure is required. Furthermore, the possibility of the nucleus or the parts that are broken off being forced through and hence rupturing the capsule is greatly if not wholly reduced. The control of the instrument as the surgeon presses the tip against the nucleus has been found to be more positive and sure than with prior hammering phaco-"emulsifiers". The control is quite positive and may, as before stated, be operated intermittently by foot pedal switch IS; and it has been found more easy for the surgeon to use than would otherwise be the case, e.g. if the tip were to be constantly rotated at high speed.

In initial tests, first with pig eyes and then with human eye, it was found, with the embodiment of Figures 2 and 3, operated at 20 kHz, that more positive control of the instrument was noticeable as the rotary oscillating slicing or cutting tip is pressed by the operator against the nucleus, than with the prior art longitudinal hammering technique; the lateral slicing or shearing fragmenting being more gentle and absent the fragment type of chipped particles broken off by such (prior art) longitudinal hammering "emulsification". Only a low 20 mm of vacuum within the tip was found needed -- not primarily to hold the nucleus against movement as in longitudinal chipping, which requires much higher holding vacuum as before stated,-- but to provide adequate aspiration of fragments.

The invention is not limited to the precise constructional details hereinbefore described and illustrated in the accompanying drawings. For example, the aspirator may be connected to a tube which surrounds the tip. The tip may also be provided with a small linear oscillating movement as before stated. The tip end may be plainly sharpened. Other means may be provided to cause lateral or rotary oscillatory movement to the tip. Dimensions, speeds and pressures may vary as required;
all such modifications being considered to fall within the scope of the invention as defined in the appended claims.

## Claims

1. A phaco-surgical ophthalmic lens nucleus fragmenting instrument comprising a longitudinally extending probe having a hollow tubular cutting tip (16) extending along the longitudinal axis thereof, means (36, 38) for setting the tip (16) into ultrasonic oscillation, means (IS) for controlling the oscillation intermittently, and means for applying an aspiration vacuum to the hollow of the tip during oscillation, characterised in that said setting means provides rotary oscillation to rotate the tip over a small angle in a plane transverse to the longitudinal axis of the probe and tip.

2. A phaco-surgical ophthalmic lens nucleus fragmenting instrument as claimed in claim 1 wherein means is provided for adding a longitudinal component of oscillation to the tip.

3. A phaco-surgical ophthalmic lens nucleus fragmenting instrument as claimed in claims 1 or 2 wherein the oscillation frequency is adjusted in the range of about 25 to 55 kHz and said small acute angle is of the order of 5°.

4. A phaco-surgical ophthalmic lens nucleus fragmenting instrument as claimed in any preceding claim wherein said vacuum is adjusted to the order of about 20 mm of Hg.

5. A phaco-surgical ophthalmic lens nucleus fragmenting instrument as claimed in any preceding claim wherein the tip is provided with a serrated cutting edge.

6. A phaco-surgical ophthalmic lens nucleus fragmenting instrument as claimed in claim 5 wherein means (I, 56, 58) is provided for introducing and withdrawing saline solution through the hollow of the tip (16).

7. A phaco-surgical ophthalmic lens nucleus fragmenting instrument as claimed in any preceding claim wherein said rotary oscillation is effected in a helical fashion.

## Patentansprüche

1. Ein phaco-chirurgisches Instrument zum Fragmentieren des Augenlinsen-Nukleus, bestehend aus einer langgestreckten Sonde, die eine hohlrohrartige Schneidspitze (16) aufweist, die sich in der Längsachse erstreckt, mit Mitteln (36, 38), um die Spitze (16) in eine Ultraschall-Oszillation zu versetzen, mit Mitteln (IS) zum Steuern der Oszillation intermittierend, und mit Mitteln, um die hohle Spitze während der Oszillation mit einem Aspirations-Vakuum zu beaufschlagen, dadurch gekennzeichnet, daß die Mittel zum Versetzen der Spitze (16) in die Ultraschall-Oszillation eine rotierende Oszillation bewirken, um die Spitze in eine Ebene quer zur Längsachse der Sonde und der Spitze um einen kleinen Winkel zu drehen oder zu schwenken.

2. Ein phaco-chirurgisches Instrument zum Fragmentieren des Augenlinsen-Nukleus nach Anspruch 1, dadurch gekennzeichnet, daß Mittel vorgesehen sind, um der Spitze eine Längskomponente der Oszillation aufzuprägen.

3. Ein phaco-chirurgisches Instrument zum Fragmentieren des Augenlinsen-Nukleus nach Anspruch 1 oder 2, dadurch gekennzeichnet, daß die Oszillationsfrequenz im Bereich zwischen etwa 25 bis 55 kHz eingestellt ist, und daß der kleine Winkel in der Größenordnung von 5° liegt.

4. Ein phaco-chirurgisches Instrument zum Fragmentierendes Augenlinsen-Nukleus nach einem der vorhergehenden Ansprüche, dadurch gekennzeichnet, daß das Vakuum auf eine Größe von ungefähr 20 mmHg eingestellt ist.

5. Ein phaco-chirurgisches Instrument zum Fragmentieren des Augenlinsen-Nukleus nach einem der vorhergehenden Ansprüche, dadurch gekennzeichnet, daß die Spitze mit einer geriffelten oder sägeartigen Schneidkante versehen ist.

6. Ein phaco-chirurgisches Instrument zum Fragmentieren des Augenlinsen-Nukleus nach Anspruch 5, dadurch gekennzeichnet, daß Mittel (I, 56, 58) zum Zuführen und Abführen einer Salzlösung durch den Hohlraum der Spitze (16) vorgesehen sind.

7. Ein phaco-chirurgisches Instrument zum Fragmentieren des Augenlinsen-Nukleus nach einem der vorhergehenden Ansprüche, dadurch gekennzeichnet, daß die rotierende Oszillation in Schraubenform erzeugt wird.

## Revendications

1. Un appareil de fragmentation du noyau d'une lentille ophtalmique phaco-chirurgicale comprenant une sonde s'étendant longitudinalement et présentant une extrémité de coupe (16) tubulaire creuse s'étendant le long de son axe longitudinal, des moyens (36, 38) pour mettre l'extrémité (16) en oscillation ultra-sonore, des moyens (IS) pour commander l'oscillation de manière intermittente, et des moyens pour appliquer un vide d'aspiration à la partie creuse de l'extrémité en cours d'oscillation, caractérisé en ce que lesdits moyens de mise en oscillation créent une oscillation rotative pour faire tourner l'extrémité sur un petit angle dans un plan transversal à l'axe longitudinal de la sonde et de l'extrémité.

2. Un appareil de fragmentation du noyau d'une lentille ophtalmique phaco-chirurgicale comme revendiqué à la revendication 1, dans lequel des moyens sont prévus pour ajouter une composante d'oscillation longitudinale à l'extrémité.

3. Un appareil de fragmentation du noyau d'une lentille ophtalmique phaco-chirurgicale comme revendiqué à la revendication 1 ou 2, dans lequel la fréquence des oscillations est réglée dans la gamme d'environ 25 à 55 kHz et ledit angle aigu est de l'ordre de 5°.

4. Un appareil de fragmentation du noyau d'une lentille ophtalmique phaco-chirurgicale tel que revendiqué dans une revendication précédente quelconque, dans lequel ledit vide est réglé pour être de l'ordre d'environ 20 mm de Hg.

5. Un appareil de fragmentation du noyau d'une lentille ophtalmique phaco-chirurgicale tel que revendiqué dans une revendication précédente quelconque, dans lequel l'extrémité est munie d'un bord de coupe dentelé.

6. Un appareil de fragmentation du noyau d'une lentille ophtalmique phaco-chirurgicale tel que revendiqué à la revendication 5, dans lequel des moyens (I, 56, 58) sont prévus pour introduire et retirer une solution saline à travers la partie creuse de l'extrémité (16).

7. Un appareil de fragmentation du noyau d'une lentille ophtalmique phaco-chirurgicale tel que revendiqué dans une revendication précédente quelconque, dans lequel ladite oscillation rotative est effectuée en hélice.
